# EUROPÄISCHE PATENTANMELDUNG

(11) **EP 1 779 861 A1**
(43) Veröffentlichungstag der Anmeldung: **02.05.2007**
(21) Anmeldenummer: 05023333.7
(22) Anmeldetag: 26.10.2005
(51) Int. Cl.: A61K 38/18, A61P 3/04

(54) **Verwendung des Nervenwachstumsfaktors (NGF) zur Herstellung eines Medikaments zur Förderung der Gewichtsabnahme**

(71) Anmelder: Staidson (Beijing) Pharmaceutical Co., LTD, Beijing Economic & Technology Zone Beijing 100176 (CN)
(72) Erfinder: Zhou, ZhiWen, Development Zone Beijing 100176 (CN); Feng, YuXia, Development Zone Beijing 100176 (CN); Zhang, HongShan, Development Zone Beijing 100176 (CN)
(74) Vertreter: Jeck, Anton

(57) **Zusammenfassung**

Der an sich bekannte Nervenwachstumsfaktor wird bei der Präparation von Medikamenten angewendet, die wirksam dazu beitragen, Körpergewicht zu verlieren.

## Beschreibung

Diese Erfindung betrifft die Anwendung eines Nervenwachstumsfaktors bei der Präparation von Medikamenten, die beim Gewichtsverlust wirksam helfen können und zum biomedizinischen Gebiet gehören.

Das Körpergewicht ist hauptsächlich durch das relative Gleichgewicht zwischen der Nahrungsaufnahme und dem Energieverbrauch bestimmt. Obwohl einige andere Faktoren wie Vererbung die Fettleibigkeit ergeben können, sind die meisten üblichen Gründe für die Gewichtszunahme oder die physikalische Fettleibigkeit eine viel zu kalorienreiche Nahrungsaufnahme und/oder ein abnehmender Energieverbrauch. Für die übergewichtigen oder dicken Menschen erhöht das exzessive Fett die Häufigkeit von kardiocerebrovaskulären oder Atmungsbeschwerden, die einen hohen Blutdruck, eine Hyperlipemia, nicht insulinabhängige Diabetes mellitus, eine koronare Herzkrankheit, ein Schlafapnöesyndrom usw. umfassen.

Die Fettleibigkeit kann durch eine geeignete Diät, eine Änderung der Lebensweise und durch eine medizinische Therapie teilweise vermindert oder verhindert werden. Die üblichen Arzneien umfassen Orlistat (kann die Fettmenge verringern, die aus der Nahrung durch den Darmtrakt absorbiert wird), Sibutramin (kann den Appetit durch Unterdrückung der Aufnahme von Noradrenalin und 5-Hydroxytryptamin zügeln) und Fenfluramin (kann den Appetit durch Freisetzung von 5-Hydroxytryptamin und einer darauf folgenden Unterdrückung der Wiederaufnahme dieser Verbindung zügeln). Aber die meisten Arzneien besitzen leichte oder ernste Nebenwirkungen, beispielsweise hat sich von Fenfluramin klinisch herausgestellt, dass es das Risiko eines kardiovaskulären Schadens bedeutend erhöht, und daher wurde es von FDA 1999 vom Markt genommen. Weitere bekannte Nebenwirkungen dieser Medikamente umfassen den Schwindelanfall, Kopfschmerzen, Herzklopfen, Schlaflosigkeit, Durchfall, Darmtraktkrampf, hohen Blutdruck usw. Zusätzlich zu diesen Nebenwirkungen machen viele Medikamente, beispielsweise Amphetamin, süchtig.

Der Nervenwachstumsfaktor (NGF) ist ein neurozytischer Regelfaktor, der in Tumorzellen von Ratten durch Rita Levi-Montalcini, die eine italienische Embriologistin war, und Stanley Cohen entdeckt wurde, der ein amerikanischer Chemiker war, wobei dieser Faktor hauptsächlich von der Zielzelle stammt, die durch das neurale Kammneuron dominiert ist. Beispielsweise gibt es reiche NGF in der zentralen Gialzelle, der peripheren Schwann-Zelle, in den Skelettmuskeln und in anderen Drüsen, in der submaxilliären Drüse der erwachsenen Ratte, in Rindersamen, im tierischen Gift und in der menschlichen Plazenta.

Über mehr als fünfzig Jahre wurde die Suche nach NGF, die von Wissenschaftlern weltweit durchgeführt wurde, tiefer und extensiver, die zeigte, dass NGF bemerkenswerte Wirkungen auf Nerventrauma, das Immunsystem und andere Systeme hatte, dass jedoch Nebenwirkungen des NGF auf Tier oder Mensch nicht festgestellt werden konnten.

Heute werden die verkauften Medikamente, deren Hauptbestandteil NGF ist, meistens zur Behandlung von Krankheiten verwendet, die beispielsweise Schäden an Hand- und Fußnerven, Alzheimer Krankheit, nervöse Gewebetransplantation. Himischemia, periphere Neuritis usw. sein können. Kein Bericht über die Behandlung der Fettleibigkeit mit NGF ist jedoch bisher erschienen.

Die Aufgabe dieser Erfindung besteht darin, eine Anwendung von NGF zu schaffen, bei der dazu beigetragen wird, dass Körpergewicht wirksam abgebaut wird.

Um diese Aufgabe zu lösen, sind die ergriffenen, technischen Protokolle folgende:
NGF wird in der Präparation von Medikamenten angewendet, die dazu beitragen können, dass ein Gewichtsverlust wirksam eintritt.

Die Untersuchungen des NGF zeigten, dass die Ratte zwei Wochen nach Verabreichung von NGF eine unbedeutende Gewichtszunahme hatte und dass diese Wirkung dosisunabhängig war. Zum Zeitpunkt von mehr als vier Wochen nach der Verabreichung von NGF dauerte das Phänomen, dass das Gewicht dasselbe war, fort, worauf wir diesen Versuch durchführten und eventuell zu dieser Erfindung kamen.

Die Vorteile dieser Erfindung sind:
1) Sie trägt wirksam zur Gewichtsverringerung bei. Im Vergleich mit vielen anderen verkauften Medikamenten, die zum Gewichtsverlust beitragen, kann NGF die Gewichtszunahme direkt steuern. Das Gewicht erhöht sich nicht wieder, wenn die Gabe von NGF abgebrochen wird.
2) Sie hat keine Nebenwirkungen. Im Vergleich mit anderen, bekannten Medikamenten hat NGF offensichtlich keine Nebenwirkung, und er macht nach unseren Untersuchungen nicht süchtig. Daher kann NGF das Gewicht wirksam verringern, und NGF verspricht gute soziale und wirtschaftliche Aussichten.

### Beispiele

### Beispiel 1

Die Verfahren dieses Versuchs bestand darin, eine Injektion oder ein nasales Sprühmittel von NGF zu verabreichen und dann die Gewichtsänderungen von Ratten zu beobachten, um das Phänomen bestätigen zu können, dass NGF eine Gewichtszunahme verhindern kann.
1. Medikament: Injektion des Nervenwachstumsfaktors, nasales Sprühmittel von NGF (geliefert von Beijing Joinn Pharmaceutical Center)
2. Tiere: 40 Wister-Ratten (gekauft vom "laboratory animal center of Military Medical Academy"); alle Tiere waren männlich und ihnen wurden Standardgetränke verabreicht. Diesen Ratten wurde die Injektion oder das nasale Sprühmittel mit NGF in einer Dosis von 4000 Einheiten/kg verabreicht. Die unbehandelte Kontrollgruppe waren Ratten, die normal aufgezogen wurden. Das Gewicht der Ratte wurde zum Zeitpunkt des Beginns der Verabreichung und jeweils nach 2, 4. 6, 10, 12, 15 Wochen im Verlauf der Verabreichung gemessen.

### Ergebnisse

**Tabelle1**

| Der Einfluss von NGF auf das Gewicht von männlichen Ratten | | | |
|---|---|---|---|
| Anzahl der Versuchswochen | Gewicht (x±SD, g/d/per) | | |
| | Kontrollgruppe | Gruppe der intramuskulären Injektion | Nasenraumgabe |
| W0 | 179.8t13.7 | 170.0±8.9 | 174.0±13.3 |
| W2 | 282.5±23.6 | 255.9±21.4** | 226.3±17.1** |
| W4 | 339.9±28.8 | 312.0±29.9** | 270.5±23.8** |
| W6 | 392.0±36.4 | 358.0±39.1** | 334.5±32.0** |
| W8 | 422.3±39.8 | 389.8±44.5* | 365.1±40.3** |
| W10 | 458.2±42.0 | 422.6±46.9* | 398.8±47.6** |
| W12 | 454.5±43.7 | 413.3±45.2 | 407.0±46.6** |
| W15 delta | 505.00±58.3 | 463.00±81.0 | 444.00±36.3 |

| | | | |
|---|---|---|---|
| n=20,*p < 0.05, **p < 0.01, im Vergleich mit der Kontrollgruppe während der entsprechenden Periode gibt es einen bedeutenden, statistischen Unterschied; □ steht für Heilungsphase, n=5. | | | |

**Tabelle 2**

| Der Einfluss von NGF auf die Gewichtserhöhung von Ratten | | | |
|---|---|---|---|
| Anzahl der Versuchswochen | Gewicht (x±SD, g/d/per) | | |
| | Kontrollgruppe | Gruppe der intramuskulären Injektion | Nasenraumgabe |
| W0~W2 | 6.16±1.49 | 5.06±1.25* | 3.08±0.79* |
| W2~W4 | 4.52±1.D95 | 4.32±1.29 | 3.40±1.74 |
| W4~W6 | 2.90±1.15 | 2.56±1.20 | 3.56±1.39 |
| W6~W8 | 2.02±2.04 | 2.12±0.62 | 2.37±0.68 |
| W8~W10 | 2.22±0.82 | 2.05±0.98 | 1.73±1.41 |
| W10~W12 | -0.61±5.32 | -0.68±1.82 | 1.15±2.77 |
| W12~W15^{□} | 1.41±0.46 | 1.51±0.76 | 2.06±0.66 |

| | | | |
|---|---|---|---|
| n=20,*p<0.05, **p<0.01_{w0-w4}, im Vergleich mit der Kontrollgruppe während der entsprechenden Periode gibt es einen bedeutenden, statistischen, Unterschied, □ steht für Heilungsphase, n = 5. | | | |

### Beispiel 2

Das Verfahren bestand darin, eine Injektion oder ein nasales Sprühmittel von NGF zu verabreichen und dann die Gewichtsänderungen von Ratten zu beobachten, um das Phänomen bestätigen zu können, dass NGF eine Gewichtszunahme verhindern kann.

### Materialien

1. Medikament: Injektion des Nervenwachstumsfaktors, nasales Sprühmittel von NGF (geliefert von Beijing Joinn Pharmaceutical Center)
2. Tiere: 40 Wister-Ratten (gekauft vom "laboratory animal center of Military Medical Academy"); alle Tiere waren männlich und ihnen wurden Standardgetränke verabreicht. Diesen Ratten wurde die Injektion oder das nasale Sprühmittel mit NGF in einer Dosis von 4000 Einheiten/kg verabreicht. Die unbehandelte Kontrollgruppe waren Ratten, die normal aufgezogen wurden. Das Gewicht der Ratte wurde zum Zeitpunkt des Beginns der Verabreichung und jeweils nach 2, 4, 6, 10, 12, 15 Wochen im Verlauf der Verabreichung gemessen.

### Ergebnisse

**Tabelle 3**

| Der Einfluss von NGF auf das Gewicht von männlichen Ratten | | | |
|---|---|---|---|
| Anzahl der Versuchswochen | Gewicht (x±SD, g/d/per) | | |
| | Kontrollgruppe | Gruppe der intramuskuläre Injektion | Nasenraumgabe |
| W0 | 170.2±10.4 | 171.5±11.2 | 163.9±11.4 |
| W2 | 227.2±11.0 | 226.8±17.1 | 203.0±14.2** |
| W4 | 256.5±10.9 | 256.8±20.8 | 234.0±15.2** |
| W6 | 276.5±17.9 | 288.0±30.9 | 265.0±23.3 |
| W8 | 290.1±26.3 | 295.4±29.1 | 278.5±26.5 |
| W10 | 295.1±21.6 | 305.5±30.2 | 293.2±30.7 |
| W12 | 306.2±25.6 | 317.8±30.1 | 300.6±27.3 |
| W15^{□} | 321.00±39.3 | 322.0±32.9 | 316.00±36.6 |

| | | | |
|---|---|---|---|
| n=20,*p < 0.05, **p < 0.01wz, im Vergleich mit der Kontrollgruppe während der entsprechenden Periode gibt es einen bedeutenden, statistischen Unterschied. □ steht für Heilungsphase, n = 5, | | | |

**Tabelle 4**

| Der Einfluss von NGF auf das Gewicht von männlichen Ratten | | | |
|---|---|---|---|
| Anzahl der Versuchswochen | Gewicht (x±SD, g/d/per) | | |
| | Kontrollgruppe | Gruppe der intramuskuläre Injektion | Nasenraumgabe |
| W0~W2 | 3.45±0.93 | 3.32±0.67 | 2.30±0.84** |
| W2~W4 | 2.25±0.91 | 2.39±1.12 | 2.39±0.80 |
| W4~W6 | 1.14±0.71 | 1.62±1.56 | 1.72±0.72* |
| W6~W8 | 0.83±0.97 | 0.50±1.81 | 1.03±0.62 |
| W8~W10 | 0.33±0.83 | 0.32±1.11 | 0.80±054* |
| W10~W12 | 1.10±1.02 | 1.19±1.10 | 0.89±1.49 |
| W12~W15^{□} | 0.78±0.38 | 0.86±0.43 | 0.92±1.05 |

| | | | |
|---|---|---|---|
| n=20,*p < 0.05, **p < 0.01_{w0 - w2}. im Vergleich mit der Kontrollgruppe während der entsprechenden Periode gibt es einen bedeutenden, statistischen Unterschied, □ steht für Heilungsphase, n = 5. | | | |

Die Injektion oder das nasale Sprühmittel des bei dieser Erfindung verwendeten Nervenwachstumsfaktors (NGF) konnte die Gewichtszunahme von Ratten verhindern, und diese Wirkung war dosisunabhängig. Keine Nebenwirkung konnte während des Versuchs oder der Heilungsphase festgestellt werden. Daher war der Nervenwachstumsfaktor ein neues Medikament, das zum Gewichtsverlust ohne irgendeine Nebenwirkung wirksam beitragen konnte.

## Patentansprüche

1. Anwendung des Nervenwachstumsfaktors bei der Präparation von Medikamenten, die wirksam dazu beitragen, Körpergewicht zu verlieren.

2. Anwendung nach Anspruch 1,
**dadurch gekennzeichnet,**
**dass** die Präparationen Injektionen und Nasalgaben umfassen.

3. Anwendung nach Anspruch 2,
**dadurch gekennzeichnet,**
**dass** die Präparationen eine Lösungsinjektion, Suspensionsinjektion, Emulsionsinjektion, ein gefriergetrocknetes Pulver für die Injektion und aseptisches Pulver für die Injektion umfassen.

4. Anwendung nach Anspruch 2,
**dadurch gekennzeichnet,**
**dass** die Präparationen ein nasales Sprühmittel, Nasentropfen, eine Nasenlösung, Nasenpackung und nasale Aerosole umfassen.
